(19) Europäisches Patentamt
European Patent Office
Office européen des brevets

(11) Veröffentlichungsnummer: **0 077 523**
**B1**

# (12) EUROPÄISCHE PATENTSCHRIFT

(45) Veröffentlichungstag der Patentschrift: **29.05.85**

(51) Int. Cl.[4]: **C 01 B 33/28,** B 01 J 29/28

(21) Anmeldenummer: **82109452.1**

(22) Anmeldetag: **13.10.82**

## (54) Zirkon- und/oder hafniumhaltige Zeolithe und Verfahren zu ihrer Herstellung sowie ihre Verwendung.

(30) Priorität: **17.10.81 DE 3141285**
**25.05.82 DE 3219494**
**25.08.82 DE 3231467**

(43) Veröffentlichungstag der Anmeldung:
**27.04.83 Patentblatt 83/17**

(45) Bekanntmachung des Hinweises auf die Patenterteilung:
**29.05.85 Patentblatt 85/22**

(84) Benannte Vertragsstaaten:
**BE DE FR GB IT NL**

(56) Entgegenhaltungen:
**DD - A - 40 952**
**DE - A - 2 017 807**
**DE - A - 2 831 631**
**DE - B - 1 160 419**
**US - A - 3 329 480**
**US - A - 3 329 482**
**US - A - 3 702 886**

**"PENTASIL FAMILY OF HIGH SILICA CRYSTALLINE MATERIALS" G.T- KOKOTAILO & W.M. MEIER; SPECIAL PUBLICATION NO. 33, CHEM. SOC., LONDON (1979)**

(73) Patentinhaber: **HOECHST AKTIENGESELLSCHAFT, Postfach 80 03 20, D-6230 Frankfurt am Main 80 (DE)**

(72) Erfinder: **Baltes, Herbert, Dr., Johannesallee 24, D-6230 Frankfurt am Main 80 (DE)**
Erfinder: **Litterer, Heinz, Dr., Albert-Schweitzer-Allee 39, D-6200 Wiesbaden (DE)**
Erfinder: **Leupold, Ernst Ingo, Dr., Am Zäunefeld 15, D-6392 Neu-Anspach (DE)**
Erfinder: **Wunder, Friedrich, Dr., Jahnstrasse 46, D-6093 Flörsheim am Main (DE)**

**EP 0 077 523 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Als Zeolithe bezeichnet man vor allem kristalline Aluminosilicate, bei denen durch eine dreidimensionale Verknüpfung von $SiO_4$- und $AlO_4$-Tetraedern regelmäßige Strukturen mit Hohlräumen und Poren entstehen. Im hydratisierten Zustand sind diese Poren und Hohlräume mit Wasser gefüllt. Dieses läßt sich ohne Beeinflussung der Kristallstruktur entfernen oder durch andere Moleküle ersetzen. Die negativen Ladungen der $AlO_4$-Tetraeder werden durch Kationen kompensiert. Diese können gegen andere positiv geladene Ionen ausgetauscht werden. Die geschilderten Eigenschaften ermöglichen die Verwendung der Zeolithe als Ionenaustauscher, Adsorbentien und Katalysatoren (D. W. Breck: Zeolite Molecular Sieves, 1974).

Zeolithe des X-, Y-, Mordenit-, Erionit- und Offretit-Typs beispielsweise besitzen als Katalysatoren für Umwandlungsreaktionen von Kohlenwasserstoffen wie Cracken, Hydrocracken oder Isomerisierungen beträchtliches technisches Interesse. Zeolithe vom Pentasil-Typ (z. B. Zeolith ZSM-5) gewinnen als Katalysatoren für die Umwandlung von Methanol zu Kohlenwasserstoffen steigende Bedeutung.

Aufgrund der zahlreichen Einsatzmöglichkeiten als Katalysatoren besteht großes Interesse an neuen Zeolithen mit spezifischen katalytischen Eigenschaften.

Beispielsweise erhält man sehr interessante Zeolithe, wenn man anstelle von Aluminium oder/und Silizium andere Elemente in das Zeolith-Gerüst einbaut. So wurden unter anderem Zeolithe der Pentasil-Reihe bekannt, die Bor (DE-OS 2 746 790), Eisen (DE-OS 2 831 611), Arsen (DE-AS 2 830 830), Antimon (DE-OS 2 830 787), Vanadin (DE-OS 2 831 631), Chrom (DE-OS 2 831 630) oder Gallium (BE-PS 882 484) auf Tetraederplätzen enthalten.

Auch wurden Titanosilicate (US-PS 3 329 481) und Zirkonosilicate (US-PS 3 329 480) mit Zeolithstruktur bekannt, bei denen aber aufgrund des Röntgenbeugungsdiagramms und der chemischen Zusammensetzung eine Pentasil-Struktur mit Sicherheit auszuschließen ist.

Gegenstand der Erfindung sind Zirkono- und/oder Hafno-Silicate bzw. -Aluminosilicate mit Pentasil-Struktur der folgenden Zusammensetzung, ausgedrückt in Molverhälnissen der Oxide:

$SiO_2 : (0-0{,}15)\ Al_2O_3 : (0{,}002-1{,}0)\ MO_2$, wobei M gleich Zirkon und/oder Hafnium ist.

Für den Begriff Pentasile gilt dabei die Definition von Kokotailo und Meier (»Pentasil family of high silicon crystalline materials« in Special Publication No. 33 of the Chemical Society London 1980). Die Pentasil-Familie umfaßt beispielsweise die synthetischen Zeolithe ZSM-5 (US-PS 3 702 886), ZSM-8 (GB-PS 1 334 243), ZSM-11 (US-PS 3 709 979) und ZSM-23 (US-PS 4 076 842).

Gegenstand der Erfindung sind vor allem Zirkono- und/oder Hafno-Silicate bzw. -Aluminosilicate mit ZSM-5-Struktur, vorzugsweise solche mit folgender Zusammensetzung, ausgedrückt in Molverhältnissen der Oxide:

$SiO_2 : (0-0{,}15)\ Al_2O_3 : (0{,}002-1{,}0)\ MO_2$, insbesondere
$SiO_2 : (0-0{,}1)\ Al_2O_3 : (0{,}01-0{,}4)\ MO_2$, wobei M gleich Zirkon und/oder Hafnium ist.

Die erfindungsgemäßen zirkon- und/oder hafniumhaltigen Zeolithe lassen sich nach den gleichen Methoden und unter Einsatz der gleichen organischen Verbindungen herstellen, wie sie auch für die Synthese des zirkon- bzw. hafniumfreien Zeolithen ZSM-5 beschrieben wurden, beispielsweise unter Verwendung von

Alkylammoniumverbindungen (US-PS 3 702 886)
Alkylaminen (US-PS 4 151 189)
Alkyldiaminen (DE-OS 2 817 576, DE-OS 2 831 334)
Alkylaminen in Gegenwart von Alkylierungsmitteln
(EP-OS 11 362, DE-AS 2 212 810)
Aminoalkoholen (GB-PS 2 023 562)
Alkoholen (DE-OS 2 935 123, US-PS 4 175 114,
EP-OS 42 225, DE-OS 2 643 929)
Ethern (EP-OS 51 741)

Vorzugsweise verwendet man Alkylammoniumverbindungen, Alkyldiamine, oder Alkylamine in Gegenwart von Alkylierungsmitteln. Unter den Alkylammoniumverbindungen sind besonders bevorzugt die Tetrapropylammoniumverbindungen, beispielsweise das Hydroxid oder eines der Halogenide. Ein besonders geeignetes Alkyldiamin ist Hexamethylendiamin.

Zur Synthese der erfindungsgmäßen Zeolithe mischt man eine oder mehrere Verbindungen aus den genannten Klassen mit Zirkon- und/oder Hafnium-Verbindungen und mit Silizium- und Natriumverbindungen und Wasser — sowie im Falle der Aluminosilicate noch zusätzlich mit Aluminiumverbindungen — und erhitzt dieses Gemisch in einem geschlossenen Gefäß. Dem Gemisch werden vorzugsweise darüber hinaus vor dem Erhitzen Impfkristalle eines Pentasils zugesetzt.

Falls man Tetrapropylammoniumverbindungen verwendet, werden die Ausgangsverbindungen im allgemeinen in folgendem Verhältnis eingesetzt, ausgedrückt in Molverhältnissen der Oxide:

$SiO_2$ : (0—0,2) $Al_2O_3$ : (0,01—1,0) $MO_2$ :
(0,01—0,5) $Na_2O$ : (0,02—1,0) $R_2O$ : (5—100) $H_2O$,

vorzugsweise im Verhältnis

$SiO_2$ : (0—0,1) $Al_2O_3$ : (0,01—0,4) $MO_2$ :
(0,02—0,3) $Na_2O$ : (0,03—0,6) $R_2O$ : (10—40) $H_2O$,

wobei M gleich Zirkon und/oder Hafnium und R gleich Tetrapropylammonium ist.

Als Silizium-, Aluminium-, Zirkon-, Hafnium- bzw. Natriumverbindungen können beispielsweise eingesetzt werden: Kieselsäuregel, Natriumsilicat, Aluminiumhydroxid, Aluminiumsulfat, Natriumaluminat, Aluminiumhalogenide, Aluminiummetahydroxid, Zirkonhalogenide, Zirkonsulfat, Zirkonylchlorid, Hafniumhalogenide, Hafniumsulfat, Natriumhydroxid, Natriumsulfat, Natriumhalogenide. Aber auch andere Verbindungen der fünf genannten Elemente eignen sich für die Herstellung der erfindungsgemäßen Zeolithe.

Das Gemisch der jeweils gewählten Verbindungen mit Wasser wird im allgemeinen 18 bis 360 Stunden, vorzugsweise 24 bis 240 Stunden lang auf eine Temperatur zwischen 100 und 200° C, vorzugsweise zwischen 130 und 170° C, in einem geschlossenen Gefäß erhitzt.

Die gebildeten Zeolithe werden in üblicher Weise, z. B. durch Filtration, isoliert, gewaschen und getrocknet. Sie können nach bekannten Methoden in die katalytisch aktiven Formen überführt werden, z. B. durch Kalzinierung und/oder Ionenaustausch (D. W. Breck, Zeolite Molecular Sieves, 1974).

Die erfindungsgemäßen Zeolithe zeichnen sich nach ihrer Überführung in die katalytisch aktive Form insbesondere aus durch eine hohe Selektivität und durch eine geringe Koksabscheidung bei der Umwandlung von Methanol in niedere Olefine. Diese Reaktion führt man beispielsweise bei Temperaturen von 350 bis 430° C und einem Wasseranteil im Methanol von 0 bis 80 Gew.-% oder mit Rohmethanol durch.

Die Erfindung soll durch die folgenden Beispiele erläutert werden, wobei die Beispiele aber in keiner Weise einschränkend sein sollen. Alle angegebenen Röntgenbeugungsdaten wurden mit einem computergesteuerten Pulverdiffraktometer D-500 der Firma Siemens aufgenommen. Es wurde Kupfer-K-$\alpha$-Strahlung verwandt.

## Beispiel 1

1,66 g Natriumaluminat (54 Gew.-% $Al_2O_3$, 41 Gew.-% $Na_2O$) und 1,48 g Natriumhydroxid werden in 20 g 20gew.-%iger wäßriger Tetrapropylammoniumhydroxid-Lösung gelöst (Lösung A). Eine weitere Lösung (Lösung B) wird hergestellt, indem man 62 g 40gew.-%iges kolloidales Kieselgel in 230 g 20gew.-%iger wäßriger Tetrapropylammoniumhydroxid-Lösung löst und diese Lösung am Rotationsverdampfer auf insgesamt 220 g einengt. Lösung A und Lösung B werden miteinander vermischt. Zu dieser Mischung werden unter intensivem Rühren 3,78 g Zirkonylchlorid $ZrOCl_2 \cdot 8\,H_2O$ gegeben. Die entstandene Suspension wird homogenisiert und in einem geschlossenen Gefäß 120 h auf 160°C erhitzt. Das entstandene Produkt wird abfiltriert, mit Wasser gewaschen und bei 120°C getrocknet. Man erhält 27,3 g erfindungsgemäßes Zirkonoaluminosilicat.

Die Röntgenbeugungsanalyse zeigt ein gut kristallines Produkt mit ZSM-5-Struktur. Die chemische Analyse des 16 Stunden bei 540°C kalzinierten Produktes zeigt folgende Zusammensetzung, ausgedrückt in Molverhältnissen der Oxide:

$SiO_2$ : 0,035 $ZrO_2$ : 0,026 $Al_2O_3$ : 0,023 $Na_2O$

## Beispiel 2

0,77 g Natriumhydroxid werden in 5 g 20gew.-%iger wäßriger Tetrapropylammoniumhydroxid-Lösung gelöst (Lösung A). Eine zweite Lösung (Lösung B) wird hergestellt, indem man 12,4 g 40gew.-%iges kolloidales Kieselgel in 45 g 20gew.-%iger Tetrapropylammoniumhydroxid-Lösung löst und diese Lösung am Rotationsverdampfer auf insgesamt 45 g einengt. Lösung A und Lösung B werden miteinander vermischt. Zu dieser Mischung werden unter intensivem Rühren 1,88 g Zirkonylchlorid $ZrOCl_2 \cdot 8\,H_2O$ in 5 ml $H_2O$ und 0,1 g Impfkristalle (aus Beispiel 1) gegeben. Die entstandene Suspension wird homogenisiert und in einem geschlossenen Gefäß 160 h auf 150°C erhitzt. Das entstandene Produkt wird abfiltriert, mit Wasser gewaschen und bei 120°C getrocknet. Man erhält 4,2 g erfindungsgemäßes Zirkonosilicat.

Das Produkt besitzt laut Röntgenbeugungsanalyse eine ZSM-5-Struktur. Die chemische Analyse des

16 Stunden bei 540° C kalzinierten Produktes zeigt folgende Zusammensetzung:

$SiO_2 : 0{,}210\ ZrO_2 : 0{,}121\ Na_2O$

Beispiel 3

Das in Beispiel 1 hergestellte Zirkono-Aluminosilicat mit ZSM-5-Struktur (kalzinierte Form) wird mit Ammoniumnitrat-Lösung ausgetauscht, zusammen mit einem Binder (Boehmit) zu Strängen gepreßt (Zeolith-Anteil 65 Gew.-%) und erneut wie in Beispiel 1 kalziniert.

In einen senkrecht angeordneten, elektrisch beheizten Rohrreaktor von 1 m Länge, der mit 250 ml dieses Katalysators gefüllt ist, dosiert man stündlich 520 ml 33gew.-%iges wasserhaltiges Methanol bei einer Temperatur von 350° C und Normaldruck. Das entstehende Reaktionsgemisch wird abgekühlt, und nach Abtrennung der kondensierbaren Anteile wird die gasförmige Phase analysiert.

Die $C_2$—$C_4$-Olefin-Selektivität ist 65% und die Selektivität zu Kohlenwasserstoffen mit mehr als 4 C-Atomen ist 14%.

Vergleichsbeispiel

Die Arbeitsweise ist wie in Beispiel 3, nur, daß statt des Zirkonoaluminosilicats ein handelsüblicher Aluminosilicat-Katalysator mit ZSM-5-Struktur eingesetzt wird.

Die $C_2$—$C_4$-Olefin-Selektivität ist 56% und die Selektivität zu Kohlenwasserstoffen mit mehr als 4 C-Atomen ist 23%.

**Patentansprüche**

1. Zirkono- und/oder Hafno-Silicate bzw. -Aluminosilicate mit Pentasilstruktur der folgenden Zusammensetzung, ausgedrückt in Molverhältnissen der Oxide:

$SiO_2 : (0-0{,}15)\ Al_2O_3 : (0{,}002-1{,}0)\ MO_2,$

wobei M gleich Zirkon und/oder Hafnium ist.

2. Zirkono- und/oder Hafno-Silicate bzw. -Aluminosilicate gemäß Anspruch 1 mit ZSM-5-Struktur.

3. Zirkono- und/oder Hafno-Silicate bzw. -Aluminosilicate nach einem der Ansprüche 1 bis 2, gekennzeichnet durch folgende Zusammensetzung, ausgedrückt in Molverhältnissen der Oxide:

$SiO_2 : (0-0{,}1)\ Al_2O_3 : (0{,}01-0{,}4)\ MO_2,$

wobei M gleich Zirkon und/oder Hafnium ist.

4. Verfahren zur Herstellung von Zirkono- und/oder Hafno-Silicaten bzw. -Aluminosilicaten nach einem der Ansprüche 1 bis 3, dadurch gekennzeichnet, daß man Zirkon- und/oder Hafniumverbindungen, sowie Silizium- und Natriumverbindungen und Wasser, sowie ggf. Aluminiumverbindungen, mit einer oder mehreren organischen Verbindungen aus der Gruppe, bestehend aus Alkylammoniumverbindungen, Alkylaminen, Alkyldiaminen, Aminoalkoholen, Alkoholen und Ethern mischt und diese Mischung in einem geschlossenen Gefäß erhitzt.

5. Verfahren nach Anspruch 4, dadurch gekennzeichnet, daß man als organische Verbindungen Alkylammoniumverbindungen einsetzt.

6. Verfahren nach Anspruch 4, dadurch gekennzeichnet, daß man als organische Verbindungen Tetrapropylammoniumverbindungen einsetzt.

7. Verfahren nach Anspruch 4, dadurch gekennzeichnet, daß man als organische Verbindungen Alkylamine in Gegenwart von Alkylierungsmitteln einsetzt.

8. Verfahren nach Anspruch 4, dadurch gekennzeichnet, daß man als organische Verbindungen Alkyldiamine einsetzt.

9. Verfahren nach einem der Ansprüche 4 bis 8, dadurch gekennzeichnet, daß man der Mischung vor dem Erhitzen Impfkristalle eines Pentasils zusetzt.

10. Verfahren zur Herstellung von Zirkono- und/oder Hafno-Silicaten bzw. -Aluminosilicaten nach einem der Ansprüche 1 bis 3, dadurch gekennzeichnet, daß man eine Mischung aus Zirkon- und/oder Hafniumverbindungen, sowie Silizium-, Natrium-, Tetrapropylammoniumverbindungen und Wasser, sowie ggf. Aluminiumverbindungen, herstellt, die folgende Zusammensetzung hat, ausgedrückt in Molverhältnissen der Oxide:

$SiO_2 : (0-0{,}2)\ Al_2O_3 : (0{,}01-1{,}0)\ MO_2 :$
$(0{,}01-0{,}5)\ Na_2O : (0{,}02-1{,}0)\ R_2O : (5-100)\ H_2O,$

wobei M gleich Zirkon und/oder Hafnium und R gleich Tetrapropylammonium ist, und diese Mischung in einem geschlossenen Gefäß erhitzt.

11. Verfahren nach Anspruch 10, dadurch gekennzeichnet, daß die zu erhitzende Mischung folgende Zusammensetzung hat, ausgedrückt in Molverhältnissen der Oxide:

$SiO_2$ : (0—0,1) $Al_2O_3$ : (0,01—0,4) $MO_2$ :
(0,02—0,3) $Na_2O$ : (0,03—0,6) $R_2O$ : (10—40) $H_2O$,

wobei M gleich Zirkon und/oder Hafnium und R gleich Tetrapropylammonium ist.

12. Verwendung von Zirkono- und/oder Hafno-Silicaten bzw. -Aluminosilicaten nach einem der Ansprüche 1 bis 3 als Katalysatoren bei der Herstellung von $C_2$- bis $C_4$-Olefinen aus Methanol.

## Claims

1. Zircono- and/or hafno-silicates or -aluminosilicates having a pentasil structure and having the following composition, expressed as molar ratio of oxides:

$SiO_2$ : (0—0.15) $Al_2O_3$ : (0.002—1.0) $MO_2$,

M being zirconium and/or hafnium.

2. Zircono- and/or hafno-silicates or -aluminosilicates according to claim 1 having a ZSM-5 structure.

3. Zircono- and/or hafno-silicates or -aluminosilicates as claimed in claim 1 or 2, having the following composition, expressed as molar ratio of oxides:

$SiO_2$ : (0—0.1) $Al_2O_3$ : (0.01—0.4) $MO_2$,

M being zirconium and/or hafnium.

4. A process for the manufacture of zircono- and/or hafno-silicates or -aluminosilicates as claimed in one of Claims 1 to 3, which comprises mixing zirconium and/or hafnium, silicon and sodium compounds and water, as well as optionally aluminum compounds with one or more organic compounds selected from the group consisting of alkylammonium compounds, alkylamines, alkyldiamines, aminoalcohols, alcohols and ethers, and heating this mixture in a closed vessel.

5. The process as claimed in Claim 4, which comprises using alkylammonium compounds as organic compounds.

6. The process as claimed in Claim 4, which comprises using tetrapropylammonium compounds as organic compounds.

7. The process as claimed in Claim 4, which comprises using alkylamines in the presence of alkylating agents as organic compounds.

8. The process as claimed in Claim 4, which comprises using alkyldiamines as organic compounds.

9. The process as claimed in one of Claims 4 to 8, which comprises adding seed crystals of a pentasil to the mixture before heating.

10. A process for the manufacture of zircono- and or hafno-silicates or aluminosilicates as claimed in one of Claims 1 to 3, which comprises preparing a mixture of zirconium and/or hafnium compounds, silicon, sodium and tetrapropylammonium compounds and water, as well as optionally aluminum compounds, which has the following composition, expressed as molar ratio of oxides:

$SiO_2$ : (0—0.2) $Al_2O_3$ : (0.01—1.0) $MO_2$ :
(0.01—0.5) $Na_2O$ : (0.02—1.0) $R_2O$ : (5—100) $H_2O$,

M being zirconium and/or hafnium, and R being tetrapropylammonium, and heating this mixture in a closed vessel.

11. The process as claimed in Claim 10, wherein the mixture to be heated has the following composition, expressed as molar ratio of oxides:

$SiO_2$ : (0—0.1) $Al_2O_3$ : (0.01—0.4) $MO_2$ :
(0.02—0.3) $Na_2O$ : (0.03—0.6) $R_2O$ : (10—40) $H_2O$,

M being zirconium and/or hafnium, and R being tetrapropylammonium.

12. Use of zircono- and/or hafno-silicates or -aluminosilicates as claimed in one of Claims 1 to 3 as catalysts for the manufacture of $C_2$—$C_4$ olefins from methanol.

## Revendications

1. Zircono- et/ou hafno-silicates ou aluminosilicates à structure pentasil, ayant la composition suivante, exprimée par les proportions molaires des oxydes:

$SiO_2 : (0-0,15)\ Al_2O_3 : (0,002-1,0)\ MO_2,$

où M est le zirconium et/ou le hafnium.

2. Zircono- et/ou hafno-silicates ou aluminosilicates selon la revendication 1, à structure ZSM-5.

3. Zircono- et/ou hafno-silicates ou aluminosilicates selon l'une des revendications 1 ou 2, caractérisés par la composition suivante, exprimée par les proportions molaires des oxydes:

$SiO_2 : (0-0,1)\ Al_2O_3 : (0,01-0,4)\ MO_2,$

où M est le zirconium et/ou le hafnium.

4. Procédé de préparation de zircono- et/ou hafno-silicates ou -aluminosilicates selon l'une des revendications 1 à 3, caractérisé en ce qu'on mélange des composés du zirconium et/ou du hafnium, ainsi que des composés du silicium et du sodium, et de l'eau, et éventuellement des composés de l'aluminium, à un ou plusieurs composés organiques appartenant au groupe comprenant les composés d'alkylammonium, les alkylamines, les alkyldiames, les aminoalcools, les alcools et les éthers, et qu'on chauffe ce mélange dans un réacteur fermé.

5. Procédé selon la revendication 4, caractérisé en ce qu'on utilise en tant que composés organiques des composés d'alkylammonium.

6. Procédé selon la revendication 4, caractérisé en ce qu'on utilise en tant que composés organiques des composés du tétrapropylammonium.

7. Procédé selon la revendication 4, caractérisé en ce qu'on utilise en tant que composés organiques des alkylamines en présence d'agents d'alkylation.

8. Procédé selon la revendication 4, caractérisé en ce qu'on utilise en tant que composés organiques des alkyldiamines.

9. Procédé selon l'une des revendications 4 à 8, caractérisé en ce qu'on ajoute au mélange, avant chauffage, des germes d'un pentasil.

10. Procédé de préparation de zircono- et/ou hafno-silicates ou-aluminosilicates selon l'une des revendications 1 à 3, caractérisé en ce qu'on prépare un mélange de composés de zirconium et/ou de hafnium, ainsi que de composés de silicium, de sodium et de tétrapropylammonium, et d'eau, et aussi, éventuellement, de composés d'aluminium, qui possède la composition suivante, exprimée par les proportions molaires des oxydes:

$SiO_2 : (0-0,2)\ Al_2O_3 : (0,01-1,0)\ MO_2 :$
$(0,01-0,5)\ Na_2O : (0,02-1,0)\ R_2O : (5-100)\ H_2O$

où M est le zirconium et/ou de hafnium et R est le tétrapropylammonium, et on chauffe ce mélange dans un réacteur fermé.

11. Procédé selon la revendication 10, caractérisé en ce que le mélange à chauffer a la composition suivante, exprimée par les proportions molaires des oxydes:

$SiO_2 : (0-0,1)\ Al_2O_3 : (0,01-0,4)\ MO_2 :$
$(0,02-0,3)\ Na_2O : (0,03-0,6)\ R_2O : (10-40)\ H_2O,$

où M est le zirconium et/ou le hafnium, et R est le tétrapropylammonium.

12. Utilisation de zircono- et/ou hafno-silicates ou -aluminosilicates selon l'une des revendications 1 à 3 en tant que catalyseurs lors de la préparation d'oléfines en $C_2$ à $C_4$ à partir de méthanol.